# EUROPÄISCHE PATENTANMELDUNG

(11) **EP 4 477 638 A1**
(43) Veröffentlichungstag der Anmeldung: **18.12.2024**
(21) Anmeldenummer: 23179200.3
(22) Anmeldetag: 14.06.2023
(51) Int. Cl.: C07C 5/25, C07C 11/02

(54) **VERFAHREN ZUR ISOMERISIERUNG VON 2,4,4-TRIMETHYLPENT-2-EN ZU 2,4,4-TRIMETHYLPENT-1-EN**

(71) Anmelder: Evonik Oxeno GmbH & Co. KG, 45772 Marl (DE)
(72) Erfinder: KUCMIERCZYK, Peter, 44628 Herne (DE); FRANKE, Robert, 45772 Marl (DE); SALE, Anna Chiara, 45657 Recklinghausen (DE); FRIDAG, Dirk, 45721 Haltern am See (DE); MARKOVIC, Ana, 45721 Haltern am See (DE); RIX, Armin Matthias, 45770 Marl (DE); TERMÜHLEN, Maren, 44135 Dortmund (DE); HARDING, Laura-Selin, 46286 Dorsten (DE); METTERNICH, Jan Benedikt, 45657 Recklinghausen (DE)
(74) Vertreter: Evonik Patent Association

(57) **Zusammenfassung**

Gegenstand der Erfindung ist ein Verfahren zur Isomerisierung von 2,4,4-Trimethylpent-2-en zu 2,4,4-Trimethylpent-1-en an einem heterogenen Katalysator auf Basis eines Zeoliths oder eines lonenaustauscherharzes.

## Beschreibung

Die vorliegende Erfindung betrifft ein Verfahren zur Isomerisierung von 2,4,4-Trimethylpent-2-en zu 2,4,4-Trimethylpent-1-en an einem heterogenen Katalysator auf Basis eines Zeoliths oder eines Ionenaustauscherharzes.

Di-isobuten ist ein technisch relevantes Erzeugnis, welches durch Dimerisierung von Isobuten gewonnen wird. Di-iso-buten besteht aus den Isomeren 2,4,4-Trimethylpent-1-en (nachfolgend auch: TMP1) und 2,4,4-Trimethylpent-2-en (nachfolgend auch: TMP2) mit einer Massenverteilung TMP1 : TMP2 von etwa 78:22 bis 81 : 19 (Gleichgewichtsverteilung). Diese Mischung kann unter anderem in Carbonylierungsverfahren zu höherwertigen Produkten umgesetzt werden. Dabei weist speziell in Carbonylierungsverfahren, wie der Methoxycarbonylierung (Reaktionsprodukt ist hier der 3,5,5-Trimethylhesansäure-methylester) oder der Hydroformylierung (Reaktionsprodukt ist hier der 3,5,5-Trimethylhexanal) das innenständige Olefin TMP2 eine deutlich geringere Reaktivität als das endständige Olefin TMP1 auf. Oftmals können Reaktionsbedingungen oder Verweilzeiten bei diesen Carbonylierungsreaktionen aufgrund der mangelnden Stabilität der Katalysatoren oder aufgrund ökonomischer Faktoren (z. B. der Reaktorgröße) nicht dahingehend angepasst werden, dass das endständige TMP2 vollständig abreagiert.

Eine direkte Verwendung des unreagierten TMP2 ist aus Gründen der Reaktivität wenig sinnvoll. Daher empfiehlt es sich das TMP2 oder einen Teil davon in die reaktivere Form TMP1 mittels Isomerisierung zu überführen, um es schlussendlich zu werthaltigen Produkten umsetzen zu können.

Verfahren zur Isomerisierung von TMP2 zu TMP1 sind in der Literatur bekannt. Diese Verfahren werden jedoch homogen katalytisch und basierend auf starken mineralischen Säuren wie bspw. H₂SO4, H₃PO₄ oder Benzolsulfonsäure (vgl. US 2,554,251 A) durchgeführt. Homogenkatalytische Reaktionssysteme haben jedoch den Nachteil, dass die Isomerisierungskatalysatoren wieder aufwändig von dem erhaltenen reinem TMP1 oder den erhaltenen TMP1/TMP2-Gemischen abgetrennt werden müssen. Erst dann könnten sie den bereits genannten Carbonylierungsverfahren zugeführt werden. Außerdem sind saure Systeme in der Regel korrosiv, sodass hochwertige Werkstoffe erforderlich sind

Die Aufgabe der vorliegenden Erfindung war deshalb die Bereitstellung eines Verfahrens zur Isomerisierung von 2,4,4-Trimethylpent-2-en zu 2,4,4-Trimethylpent-1-en, welches die vorgenannten Probleme nicht aufweist. Gleichzeitig sollte mit dem Verfahren jedoch auch hohe Umsätze erzielt werden können.

Diese Aufgabe konnte durch das erfindungsgemäße Verfahren nach dem unabhängigen Anspruch 1 gelöst werden. Bevorzugte Ausführungsformen sind in den abhängigen Ansprüchen angegeben.

Erfindungsgemäß ist das Verfahren ein Verfahren zur Isomerisierung von 2,4,4-Trimethylpent-2-en zu 2,4,4-Trimethylpent-1-en, wobei das Verfahren zumindest die folgenden Schritte umfasst:
a. Bereitstellen eines Einsatzstroms, enthaltend 2,4,4-Trimethylpent-2-en und 2,4,4-Trimethylpent-1-en, wobei der Anteil von 2,4,4-Trimethylpent-2-en am Gesamtstrom mindestens 25 Mol%, bezogen auf den gesamten Einsatzstrom, beträgt;
b. Durchführung der Isomerisierung mit dem Einsatzstrom unter Verwendung eines heterogenen Katalysators auf Basis eines Zeoliths oder eines Ionenaustauscherharzes,
wobei der Anteil des 2,4,4-Trimethylpent-2-ens im Produktstrom geringer und der Anteil des 2,4,4-Trimethylpent-1-ens im Produktstrom höher ist als im Einsatzstrom.

Der Vorteil des erfindungsgemäßen Verfahrens ist die Verwendung eines heterogenen Katalysatorsystems. Ein solches Katalysatorsystem muss nicht vom Produktstrom abgetrennt werden, sondern verbleibt im Reaktionsgefäß bzw. dem Reaktor. Mit den erfindungsgemäßen Katalysatorsystemen auf Basis eines Zeoliths oder eines Ionenaustauscherharzes können zudem hohe Umsätze erzielt werden.

Der einzusetzende Einsatzstrom enthält 2,4,4-Trimethylpent-2-en und 2,4,4-Trimethylpent-1-en, wobei der Anteil von 2,4,4-Trimethylpent-2-en am Gesamtstrom mindestens 25 Mol%, vorzugsweise mindestens 28 Mol% bezogen auf den gesamten Einsatzstrom enthält. Ströme mit weniger Trimethylpent-2-en sind so nah an der Gleichgewichtsverteilung, dass sich ein Einsatz nicht lohnt. Derartige Ströme können Di-isobutenströme sein, welche mittels Dimerisierung aus Isobuten oder Isobutenhaltigen Kohlenwasserstoffgemischen hergestellt werden können, beispielsweise so wie es in EP 1 360 160 B1 offenbart wird. Weiterhin können die hier einzusetzenden Ströme als nicht reagierte Restströme bei Carbonylierungsverfahren anfallen, beispielsweise bei der Methoxycarbonylierung oder bei der Hydroformylierung.

Die Isomerisierung in Schritt b. kann grundsätzlich in jedem geeigneten Reaktor durchgeführt werden. Möglich ist, dass die Isomerisierung in einem einzigen Reaktor oder in mehreren parallel oder in Reihe geschalteten Reaktoren erfolgt. Möglich ist zudem die Durchführung in Chargen oder im kontinuierlichen Betrieb. Bevorzugt wird die Isomerisierung in einem oder mehreren kontinuierlich arbeitenden Reaktoren, die üblicherweise bei Feststoff/Flüssigkeits-Kontaktreaktionen zum Einsatz gelangen, durchgeführt. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Weiterhin ist es möglich, den Reaktor unter Produktrückführung oder im geraden Durchgang zu betreiben. Ein anderes Konzept als Festbettreaktoren sind beispielsweise Reaktoren, in denen der Ionenaustauscher oder Zeolith suspendiert in einer flüssigen Phase vorliegt.

Als Reaktoren werden bei der erfindungsgemäßen Isomerisierung bevorzugt Rohrreaktoren oder Rohrbündelreaktoren, insbesondere solche mit inneren Rohrdurchmessern von 10 bis 60 mm, verwendet. Das Verhältnis von Länge zu Durchmesser der Katalysatorschüttung kann dabei variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden.

Die Beheizung der Rohre des Reaktors, sei es Rohrreaktor oder Rohrbündelreaktor, kann mittels Dampf oder Wärmeträgeröle über den Reaktormantel erfolgen. Insbesondere bei Einsatz von flüssigen Heizmedien wird dabei die Mantelseite konstruktiv so ausgeführt, dass ein möglichst homogener Temperaturgradient an allen Rohren anliegt. Die hierfür notwendigen technischen Maßnahmen sind dem Fachmann bekannt und in der Literatur beschrieben (Einbau von Umlenkblechen, Disc- an Donut-Bauweise, Einspeisung / Abfuhr des Wärmeträgers an verschiedenen Stellen des Reaktors usw.). Bevorzugt werden Reaktionsmedium und Wärmeträger im Gleichstrom, besonders bevorzugt von oben nach unten durch die Traktorrohre bzw. den Reaktormantel geführt. Eine bevorzugte Ausführungsform ist beispielsweise in DE 10 2006 040 433 A1 beschrieben.

Die Isomerisierung von 2,4,4-Trimethylpent-2-en zu 2,4,4-Trimethylpent-1-en erfolgt exotherm, verläuft also unter Freisetzung von Energie, was zu einer Erwärmung der Reaktionsmischung führt. Zur Begrenzung des Temperaturanstiegs ist die Verdünnung des Einsatzstroms, beispielsweise durch Rückführung von Produkt möglich.

Der oder die bei der Isomerisierung eingesetzten Reaktor(en) können adiabatisch, polytrop oder praktisch isotherm betrieben werden. Praktisch isotherm bedeutet, dass die Temperatur an einer beliebigen Stelle im Reaktor maximal um 10°C höher ist als die Temperatur am Reaktoreingang. Bei adiabatischem Betrieb der Reaktoren ist es in der Regel sinnvoll, mehrere Reaktoren in Reihe zu schalten und zwischen den Reaktoren eine Kühlung vorzusehen. Reaktoren, die für einen polytropen oder praktisch isothermen Betrieb geeignet sind, sind beispielsweise die bereits erwähnten Rohrbündelreaktoren, aber auch Rührkessel- und Schlaufenreaktoren.

Das Verfahren kann bei eher milden Temperaturen durchgeführt werden. Die Isomerisierung in Schritt b wird vorzugsweise bei einer Temperatur von 25 bis 90 °C, vorzugsweise 30 bis 80 °C, besonders bevorzugt 35 bis 70 °C durchgeführt.

Weiterhin kann die erfindungsgemäße Isomerisierung bei einem Druck gleich oder größer als der Dampfdruck des Einsatzstromgemisches und/oder des Reaktionsgemisches bei der jeweiligen Reaktionstemperatur durchgeführt werden, vorzugsweise bei einem Druck von weniger als 40 bar.

Weiterhin bevorzugt wird die Isomerisierung in Schritt b. in der Flüssigphase durchgeführt. Es sollte klar sein, dass in diesem Fall Druck und Temperatur so zu wählen sind, dass der Einsatzstrom in der flüssigen Phase vorliegt bzw. vorliegen kann.

Der oder die Reaktoren können bei der erfindungsgemäßen Isomerisierung auch mehrere Katalysatoren auf Basis eines Zeoliths oder eines Ionenaustauscherharzes enthalten. So kann beispielsweise ein Gemisch von lonenaustauscherharzen unterschiedlicher Reaktivität eingesetzt werden. Ebenso ist es möglich, dass ein Reaktor Katalysatoren mit unterschiedlicher Aktivität enthält, die beispielsweise in Schichten angeordnet sind. Wird mehr als ein Reaktor verwendet, können die einzelnen Reaktoren mit dem gleichen oder unterschiedlichen Katalysator(en) auf Basis eines Zeoliths oder eines Ionenaustauscherharzes gefüllt sein.

Als heterogener Katalysator wird bei der erfindungsgemäßen Isomerisierung ein Katalysator auf Basis eines Zeoliths oder eines Ionenaustauscherharzes eingesetzt. Entsprechende Zeolithe und lonenaustauscherharze sind zahlreich im Handel verfügbar und dem Fachmann bekannt. Es hat sich gezeigt, dass der Katalysator auf Basis eines Zeoliths vorzugsweise ein Si : AI Verhältnis im Bereich von 40 : 1 bis 200 : 1 aufweist. Weiterhin hat sich gezeigt, dass als Katalysator auf Basis eines Ionenaustauscherharzes vorzugsweise der Styrol-Divinylbenzol-Typ als H-Form und in teilneutraliserter Form gut geeignet ist.

Ist der Katalysator ein Zeolith, haben sich einige Zeolithe als besonders vorteilhaft herausgestellt, beispielsweise beta- und gamma-Zeolithe. Der Zeolith wird daher vorzugsweise aus der Gruppe bestehend aus Z-beta-H-25, Z-beta-H-38, Z-beta-H-360, Z-Mor-H-20, Z-Y-H-60, Z-Y-H-80, Z-beta-H, Z-CFG-1, Z-beta-ammonium-38, Z-CBV 760 CY (1.6), Z-CBV 780 CY (1.6), CP 814E CY (1.6), CBV 500 CY (1.6), H-CZB-150 und Mischungen davon ausgewählt.

Als lonenaustauscherharz können beispielsweise lonenaustauscherharze eingesetzt werden, solche, die durch die Sulfonierung von Phenol/Aldehyd-Kondensaten oder durch die Sulfonierung von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol.

Besonders bevorzugt werden lonenaustauscherharze für die Isomerisierung eingesetzt, die durch die Sulfonierung von Cooligomeren, die durch Umsetzung von Styrol mit Divinylbenzol entstehen, hergestellt werden. Die lonenaustauscherharze können gelförmig, makroporös oder schwammförmig hergestellt werden. Die Eigenschaften dieser Harze, insbesondere spezifische Oberfläche, Porosität, Stabilität, Quellung oder Schrumpfung und Austauschkapazität, können bekanntermaßen durch den Herstellprozess variiert werden.

Ionenaustauscherharze des bevorzugten Styrol-Divinylbenzol-Typs werden u. a. unter folgenden Handelsnamen verkauft: CT 151 und CT275 der Firma Purolite, Amberlyst^{®} 15, Amberlyst^{®} 35, Amberlite^{®} IR-120, Amberlite^{®} 200 der Firma Rohm&Haas, Dowex M-31 der Firma DOW, LEWATIT^{®} K 2621, LEWATIT^{®} K 2431 der Firma Lanxess.

Das Porenvolumen der als Katalysatoren einsetzbaren lonenaustauscherharze, insbesondere des bevorzugten Styrol-Divinylbenzol-Typs, beträgt vorzugsweise 0,3 bis 0,9 ml/g, besonders bevorzugt 0,5 bis 0,9 ml/g. Das Porenvolumen kann beispielsweise mittels adsorptiver Techniken ermittelt werden. Die Korngröße der lonenaustauscherharze beträgt bevorzugt van 0,3 mm bis 1,5 mm, besonders bevorzugt 0,5 mm bis 1,0 mm. Die Korngrößenverteilung kann enger oder weiter gewählt werden. So können beispielsweise lonenaustauscherharze mit sehr einheitlicher Korngröße (monodisperse Harze) eingesetzt werden.

Die als Katalysatoren bei der Isomerisierung einsetzbaren lonenaustauscherharze können als teilneutralisierte lonenaustauscherharze vorliegen. Dazu kann das lonenaustauscherharz mit Sären oder Basen behandelt werden, wie es in der EP 1 360 160 B1 beschrieben wird.

Aus der Isomerisierung in Schritt b. wird dann ein Produktstrom erhalten, bei dem der Anteil des 2,4,4-Trimethylpent-2-ens geringer und der Anteil des 2,4,4-Trimethylpent-1-ens im Produktstrom höher ist als im Einsatzstrom. In einer bevorzugten Ausführungsform der vorliegenden Erfindung beträgt der Anteil von 2,4,4-Trimethylpent-1-en im Produktstrom von 60 bis 80 Mol%, vorzugsweise 65 bis 80 Mol%. Der Anteil von 2,4,4-Trimethylpent-2-en im Produktstrom beträgt dann 20 bis 40 Mol%, vorzugsweise 20 bis 35 Mol%.

Die Erfindung wird nachfolgend anhand von Beispielen beschrieben. Diese dienen der Erläuterung und stellen keine Einschränkung des Erfindungsgegenstands dar.

### Beispiel

### Beispiel 1

2,4 g eines erfindungsgemäßen Katalysators (Purolite CT 275, teilneutralisiert, 0,9 eq/L) wurden in einem Rohrreaktor mit einem Durchmesser von 0,6 cm eingefüllt. Der Reaktor wurde mit 2,4,4-trimethylpten-2-en (>99%) beschickt. Die Isomerisierung erfolgte bei einer Temperatur von 55 °C und Umgebungsdruck. Nach Austritt aus dem Reaktor wurde die Verteilung von 2,4,4Trimethylpent-2-en zu 2,4,4-Trimethylpent-1-en bestimmt (im Eingangsstrom 2,4,4Trimethylpent-2-en : 2,4,4-Trimethylpent-1-en 99 : 1). Die Analyse erfolgte durch Gaschromatographie. Ausgewertet wurden die Peak-Flächen nach Methode der externen Kalibration.

| Massen-Strom 2,4,4-trimethylpent-2-en [g/min] | Temperatur [°C] | w-Anteil 2,4,4-trimethylpent-2-en an Di-iso-buten-Isomeren im Produktstrom [%] | w-Anteil 2,4,4-trimethylpent-1-en an Di-iso-buten-Isomeren im Produktstrom [%] |
|---|---|---|---|
| 0.1 | 55 | 22^{a} | 78^{a} |

| | | | |
|---|---|---|---|
| ^{a}Über 80 Stunden Versuchszeit. | | | |

Die Ergebnisse zeigen, dass der erfindungsgemäße Katalysator sehr gut für die Isomerisierung geeignet ist.

### Beispiel 2

2,0 g eines erfindungsgemäßen Katalysators (CBV760) wurden in einem Rohrreaktor mit einem Durchmesser von 0,6 cm eingefüllt. Der Reaktor wurde mit 2,4,4-trimethylpten-2-en (>99%) beschickt. Dabei wurde das 2,4,4-Trimethylpent-2-en mit einem Volumenströmen von 0,12 g/min durch den Reaktor geleitet. Die Isomerisierung erfolgte bei einer Temperatur von 55°C und Umgebungsdruck. Nach Austritt aus dem Reaktor wurde die Verteilung von 2,4,4Trimethylpent-2-en zu 2,4,4-Trimethylpent-1-en bestimmt (im Eingangsstrom 2,4,4Trimethylpent-2-en : 2,4,4-Trimethylpent-1-en 99 : 1). Die Analyse erfolgte durch Gaschromatographie. Ausgewertet wurden die Peak-Flächen nach Methode der externen Kalibration.

| Massen-Strom 2,4,4-trimethylpent-2-en [g/min] | Temperatur [°C] | w-Anteil 2,4,4-trimethylpent-2-en an Di-iso-buten-Isomeren im Produktstrom [%] | w-Anteil 2,4,4-trimethylpent-1-en an Di-iso-buten-Isomeren im Produktstrom [%] |
|---|---|---|---|
| 0.126 | 55 | 27^{a} | 73^{a} |

| | | | |
|---|---|---|---|
| ^{a}Über 24 Stunden Versuchszeit. | | | |

Die Ergebnisse zeigen, dass der erfindungsgemäße Katalysator sehr gut für die Isomerisierung geeignet ist.

## Patentansprüche

1. Verfahren zur Isomerisierung von 2,4,4-Trimethylpent-2-en zu 2,4,4-Trimethylpent-1-en, wobei das Verfahren zumindest die folgenden Schritte umfasst:
a. Bereitstellen eines Einsatzstroms, enthaltend 2,4,4-Trimethylpent-2-en und 2,4,4-Trimethylpent-1-en, wobei der Anteil von 2,4,4-Trimethylpent-2-en am Gesamtstrom mindestens 25 Mol%, bezogen auf den gesamten Einsatzstrom, beträgt;
b. Durchführung der Isomerisierung mit dem Einsatzstrom unter Verwendung eines heterogenen Katalysators auf Basis eines Zeoliths oder eines Ionenaustauscherharzes,
wobei der Anteil des 2,4,4-Trimethylpent-2-ens im Produktstrom geringer und der Anteil des 2,4,4-Trimethylpent-1-ens im Produktstrom höher ist als im Einsatzstrom.

2. Verfahren nach Anspruch 1 oder 2, wobei Anteil von 2,4,4-Trimethylpent-1-en im Produktstrom von 60 bis 80 Mol%, vorzugsweise 65 bis 80 Mol% beträgt.

3. Verfahren nach einem der Ansprüche 1 bis 3, wobei Anteil von 2,4,4-Trimethylpent-2-en im Produktstrom von 20 bis 40 Mol%, vorzugsweise 20 bis 35 Mol% beträgt.

4. Verfahren nach einem der vorhergehenden Ansprüche, wobei die Isomerisierung in Schritt b bei einem Druck von weniger als 40 bar durchgeführt wird.

5. Verfahren nach einem der vorhergehenden Ansprüche, wobei die Isomerisierung in Schritt b bei einer Temperatur von 25 bis 90 °C, vorzugsweise 30 bis 80 °C, besonders bevorzugt 35 bis 70 °C durchgeführt wird.

6. Verfahren nach einem der vorhergehenden Ansprüche, wobei das Zeolith ein Si : AI Verhältnis im Bereich von 40 : 1 bis 200 : 1 aufweist.

7. Verfahren nach einem der vorhergehenden Ansprüche, wobei das Zeolith aus der Gruppe der beta- und gamma-Zeolithe ausgewählt wird.

8. Verfahren nach einem der vorhergehenden Ansprüche, wobei der Katalysator ein Zeolith ist und aus der Gruppe bestehend aus Z-beta-H-25, Z-beta-H-38, Z-beta-H-360, Z-Mor-H-20, Z-Y-H-60, Z-Y-H-80, Z-beta-H, Z-CFG-1, Z-beta-ammonium-38, Z-CBV 760 CY (1.6), Z-CBV 780 CY (1.6), CP 814E CY (1.6), CBV 500 CY (1.6), H-CZB-150 und Mischungen davon ausgewählt wird.

9. Verfahren nach einem der vorhergehenden Ansprüche, wobei als lonenaustauscherharz solche eingesetzt werden, die durch die Sulfonierung von Phenol/Aldehyd-Kondensaten oder durch die Sulfonierung von Cooligomeren von aromatischen Vinylverbindungen hergestellt werden.

10. Verfahren nach einem der vorhergehenden Ansprüche, wobei die aromatischen Vinylverbindungen zur Herstellung der Cooligomere aus der Gruppe bestehend aus Styrol, Vinyltoluol, Vinylnaphthalin, Vinylethylbenzol, Methylstyrol, Vinylchlorbenzol, Vinylxylol und Divinylbenzol, ausgewählt werden.

11. Verfahren nach einem der vorhergehenden Ansprüche, wobei als lonenaustauscherharz solche eingesetzt werden, die durch die Sulfonierung von Cooligomeren, die durch Umsetzung von Styrol mit Divinylbenzol entstehen, hergestellt werden.

12. Verfahren nach einem der vorhergehenden Ansprüche, wobei als lonenaustauscherharz teilneutralisierte lonenaustauscherharze eingesetzt werden.

13. Verfahren nach einem der vorhergehenden Ansprüche, wobei die Isomerisierung in der Flüssigphase durchgeführt wird.
